# EUROPÄISCHE PATENTSCHRIFT

(11) **EP 0 906 769 B1**
(45) Veröffentlichungstag und Bekanntmachung des Hinweises auf die Patenterteilung: **30.06.2004**
(21) Anmeldenummer: 98116921.2
(22) Anmeldetag: 08.09.1998
(51) Int. Cl.: A61N 5/10

(54) **Flexibler Körper mit darin eingebetteten Schläuchen oder Kathetern, insbesondere für die Strahlentherapie sowie Verfahren zu seiner Herstellung**
Flexible body with inside tubes or catheters,particularly suited for the therapy by rays,and its manufacturing process
Corp flexible comprenant en son sein des tubes ou des cathéters,destinés en particulier à la thérapie par rayons ainsi que son procédé de fabrication

(30) Priorität: 04.10.1997 DE 19743877
(43) Veröffentlichungstag der Anmeldung: 07.04.1999
(73) Patentinhaber: KABE Labortechnik Gesellschaft mit beschränkter Haftung, D-51588 Nümbrecht-Elsenroth (DE)
(72) Erfinder: Kolpe, Dieter, 51674 Wiehl (DE)
(74) Vertreter: Christophersen & Partner

(56) Entgegenhaltungen:
- EP-A- 0 754 473
- DE-A- 4 342 589
- DE-A- 19 526 690
- US-A- 4 706 652

## Beschreibung

Die Erfindung betrifft zunächst ein Verfahren zur Herstellung eines flexiblen Körpers mit darin eingebetteten Schläuchen oder Kathetern, insbesondere für die Strahlentherapie im Nachladeverfahren (After-Loading), bei dem die Schläuche oder Katheter in eine Form eingelegt und darin in definierter Lage und Ausrichtung fixiert werden, bevor zur Ausformung des Grundmaterials des Körpers eine flüssige oder gelartige Masse in den Formraum der Form eingefüllt wird und darin unter Einbettung der Schläuche oder Katheter aushärtet.

Derartige flexible Körper, oft auch als "Flabs" bezeichnet, dienen in der Medizintechnik dazu, auf kleinstem Raum konzentrierte Strahlenbehandlungen hoher Wirksamkeit durchzuführen. Die Strahlentherapie nach der After-Loading-Methode hat sich insbesondere bei der Behandlung von Tumoren bewährt. Zur Vermeidung einer Schädigung des gesunden Gewebes ist bei derartigen Therapien eine sehr genaue örtliche Planung der Bestrahlung sicherzustellen, weshalb die Katheter genau positioniert am Körper oder im körpereigenen Gewebe fixiert werden müssen.

Ein Verfahren zur Herstellung derartiger Flabs ist z. B. aus der DE 195 26 690 A1 bekannt. In eine rechteckige, zweigeteilte Form werden zunächst über deren gesamte Länge die später einzubettenden, röhrchenförmigen Katheter eingesetzt. Anschließend wird eine Geliermasse in die Form gefüllt, so daß diese beim Aushärten die Katheter umschließt und hierbei einbettet. Zusätzlich kann ein Gewebematerial in das Material des so erzeugten Blockes mit eingebettet werden.

Während infolge der Einbettung der Verlauf der einzelnen Katheter sehr genau identifizierbar und während der Strahlentherapie reproduzierbar ist, bereitet die Identifikation eines bestimmten Applikationsortes in Längsrichtung der jeweiligen Katheter Schwierigkeiten.

Der Erfindung liegt die **Aufgabe** zugrunde, die bekannten flexiblen Körper mit darin eingebetteten Schläuchen oder Kathetern so weiterzuentwickeln, daß bei deren Anwendung in der Strahlentherapie eine exaktere Positionierung der Strahlenquelle ermöglicht wird. Ferner soll ein Verfahren zur Herstellung derartiger flexibler Körper entwickelt werden.

Zur **Lösung** wird hinsichtlich des Verfahrens vorgeschlagen, daß die Schläuche oder Katheter in der Weise in die Form eingelegt werden, daß sich deren eine Enden noch innerhalb des Formraums der Form befinden, daß diese Enden dauerhaft verschlossen und daran vor Einfüllen der flüssigen oder gelartigen Masse Ausrichtelemente angesetzt werden, die sich an der Form abstützen.

Die so hergestellten Flabs weisen daher keine durchgehenden Schläuche bzw. Katheter auf, sondern sind noch im Grundmaterial des Körpers mit Begrenzungen versehen. Diese Begrenzungen stellen in Längsrichtung jedes einzelnen Katheters exakt reproduzierbare Orte dar, die als Bezugspunkte bei der Festlegung der Ortskoordinaten des jeweils erforderlichen Applikationsortes dienen können. Auf diese Weise ist eine exakte Ortsbestimmung nicht nur quer zur Erstreckung der Katheter möglich, wozu der geeignete der parallel angeordneten Katheter ausgewählt wird, sondern auch in Längsrichtung des jeweiligen Katheters.

Vorteilhafte Ausgestaltungen des Verfahrens sind in den Unteransprüchen beschrieben.

Hinsichtlich der Teilaufgabe, einen entsprechenden flexiblen Körper zu schaffen, wird mit der Erfindung vorgeschlagen ein flexibler Körper mit darin eingebetteten Schläuchen oder Kathetern, insbesondere für die Strahlentherapie im Nachladeverfahren (After-Loading), in dem sich die einen Enden der Schläuche oder Katheter innerhalb des Körpers befinden und dort durch in die Enden eingesetzte Stopfen (3) verschlossen sind, wobei die nach außen weisende Stimfläche des Stopfens (3) durch das Grundmaterial (1) des flexiblen Körpers abgedeckt ist.

Auch zum Aufbau des flexiblen Körpers sind vorteilhafte Ausgestaltungen in den Unteransprüchen angegeben.

Weitere Einzelheiten und Ausgestaltungen werden nachfolgend anhand der Zeichnung erläutert. Darin zeigen:
- Fig. 1: in einer Ansicht einen erfindungsgemäß ausgebildeten flexiblen Körper ("Flab"),
- Fig. 2: einen vergrößerten Teilausschnitt entlang der Ebene II-II der Fig. 1,
- Fig. 3: in einer Schnittdarstellung eine Form zur Herstellung des flexiblen Körpers,
- Fig. 4: in einer Draufsicht die Form nach Fig. 3 in geschlossenem Zustand,
- Fig. 5: in einer Stirnansicht die Form nach Fig. 3,
- Fig. 6: in vergrößerter Darstellung die Einzelheit VI gemäß Fig. 3,
- Fig. 7: in einer der Fig. 3 entsprechenden Schnittdarstellung die Form zur Herstellung des flexiblen Körpers in einem späteren Verfahrensstadium,
- Fig. 8: in einer Draufsicht die Form nach Fig. 7 und
- Fig. 9: in einer vergrößerten Darstellung die Einzelheit IX gemäß Fig. 7.

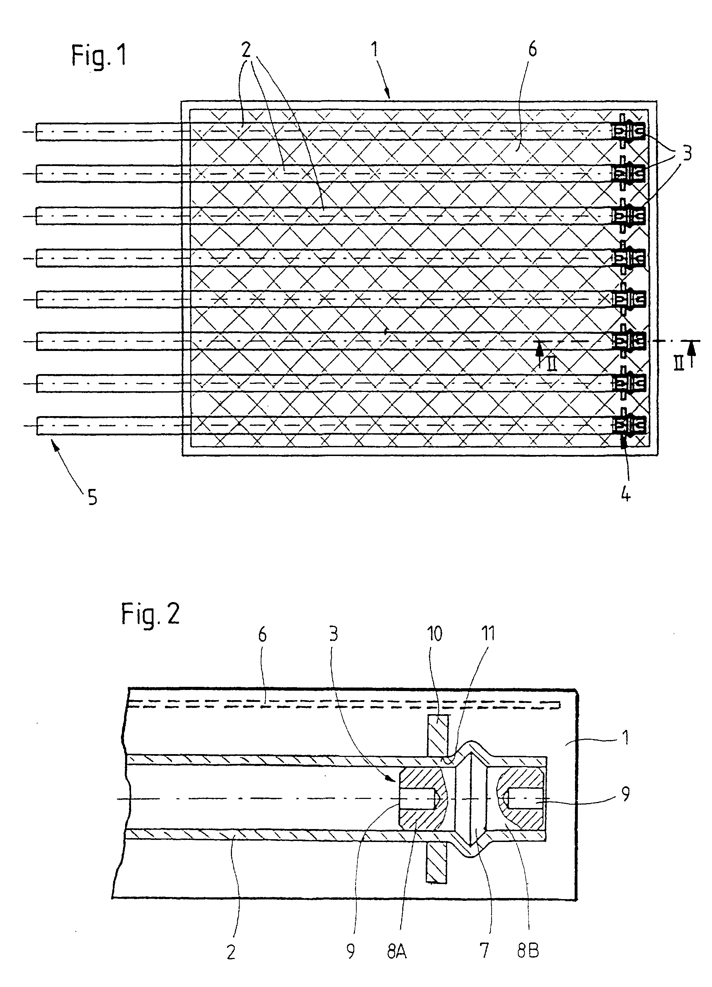

Der in Fig. 1 und vergrößert in Fig. 2 dargestellte flexible Körper ("Flab") besteht aus einem blockförmigen Grundmaterial 1, in welches mehrere schlauchförmige Katheter 2 eingesetzt sind. Mittels Stopfen 3 sind die sich innerhalb des Grundmaterials 1 befindenden Enden 4 der Katheter 2 verschlossen. Die jeweils anderen Enden 5 führen aus dem Grundmaterial 1 heraus und sind offen, so daß sich hierdurch eine Strahlungsquelle bis in das Innere des jeweiligen Katheters 2 einführen läßt.

Der im Ausführungsbeispiel erläuterte Körper ist als flacher Quader geformt, in den die einzelnen Katheter 2 in einer gemeinsamen Ebene und parallel zueinander eingebettet sind. Je nach den therapeutischen Randbedingungen sind auch andere Gestaltungen des Körpers oder andere Anordnungen der Katheter 2 darin möglich, z. B. auch die Formung des Körpers in Gestalt eines Zylinders.

In das Grundmaterial 1 des Körpers mit eingebettet ist eine Gewebeeinlage 6, die den flexiblen Körper, der im Wege einer Operation in den Körper des Patienten implantiert wird, hinsichtlich der Zugkräfte zu verstärken. Bei der Strahlenbehandlung muß sich die zu bestrahlende Stelle im Körper des Patienten auf der der Gewebeeinlage 6 abgewandten Seite des flexiblen Körpers befinden.

Zur Bereitstellung der erforderlichen Flexibilität des Körpers besteht dieser aus einem vorzugsweise transparenten, bei Erhitzung gießfähigen Kunststoffmaterial. In Betracht zu ziehen sind insbesondere körperfreundliche, weiche Kunststoffe auf Basis von Polyurethanen, Polyolefinen, Polycarbonaten, Polyestern, Polyamiden, Polyethern, Polyvinylchlorid, Polysulfonen und entsprechenden Polymeren und Silikonen mit oder ohne Weichmachern. Die Katheter 2 können beispielsweise aus Polyamid bestehen. Das Kunststoffgewebe für die Gewebeeinlage 6 sollte ebenfalls das Kriterium der Körperfreundlichkeit erfüllen, da dieses bei dem Implantieren des Flabs mit dem Körpergewebe des Patienten vernäht werden muß.

Einzelheiten im Bereich der verschlossenen Enden der Katheter werden nachfolgend anhand der Fig. 2 erläutert. Der symmetrisch gestaltete Stopfen 3 aus Kunststoff setzt sich aus einem radial erweiterten Mittelabschnitt 7 sowie zwei Endabschnitten 8A, 8B zusammen. Die Endabschnitte 8A, 8B sind an ihren Stirnseiten mit Sacklöchern 9 versehen, die als Elemente zum Verbinden des Stopfens 3 mit anderen Teilen dienen, wie nachfolgend noch näher erläutert werden wird. Der äußere Durchmesser der Endabschnitte 8A, 8B entspricht in etwa dem Innendurchmesser des Katheters 2, der außen gelegene Endabschnitt 8B schließt ferner im wesentlichen bündig mit dem dortigen Ende 4 des Katheters 2 ab.

Im Bereich des nach innen weisenden Endabschnittes 8A des Stopfens 3 sitzt auf der Mantelfläche des Katheters 2 ein Klemmring 10 auf. Beim Ausführungsbeispiel ist jeder einzelne Katheter 2 mit einem Klemmring 10 versehen, es ist jedoch gleichfalls möglich, alle oder einen Teil der Klemmringe zu einem gemeinsamen Klemmstück zusammenzufassen, in dem sich dann die einzelnen Bohrungen 11 zur Durchführung der Katheter 2 befinden. Auch die Klemmringe 10 bzw. das gemeinsame Klemmstück besteht aus Kunststoff.

Um das verschlossene Ende 4 der Katheter 2 herum befindet sich ebenfalls das Grundmaterial 1, so daß der Körper bis auf die freien Öffnungen der Katheter vollkommen abgeschlossen ist.

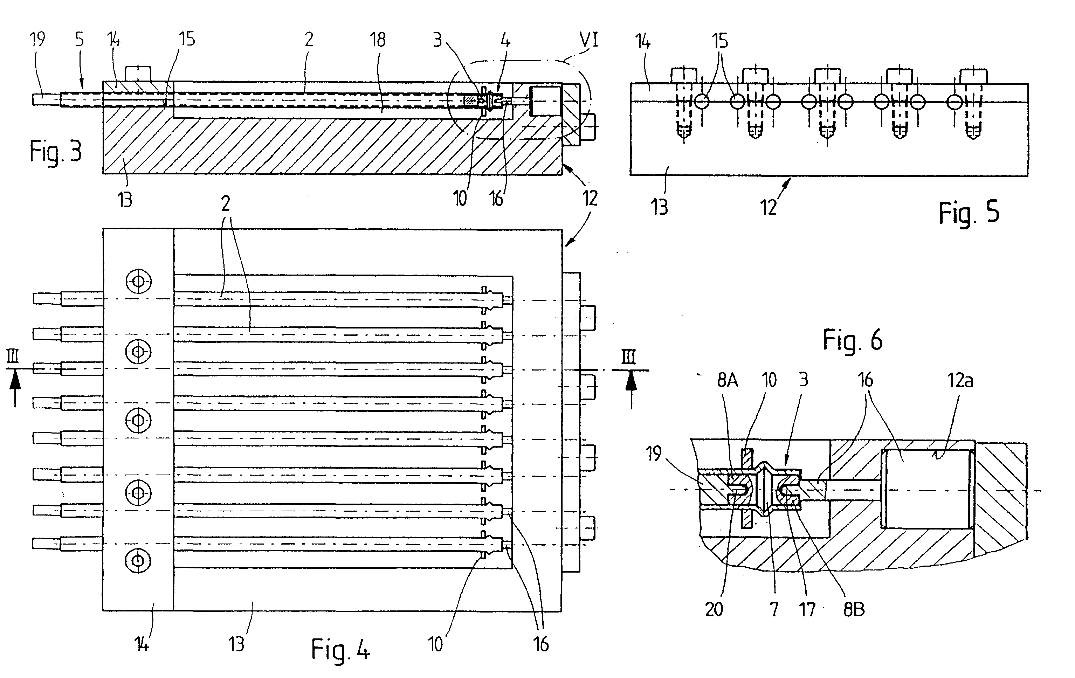

Anhand der Figuren 3 bis 9 wird nunmehr die Herstellung des Körpers erläutert. Hierzu wird eine zweiteilige Form 12 verwendet, die sich aus einem Grundkörper 13 und einem auf dem Grundkörper 13 befestigbaren Klemmstück 14 zusammensetzt. Grundkörper 13 und Klemmstück 14 sind an ihrem einen Ende mit halbkreisförmigen Durchführungen versehen, die sich zusammengesetzt zu Bohrungen 15 fügen, in denen einzeln die Katheter 2 mit ihren freien Enden 5 festgespannt sind. Im Bereich der anderen Enden 5 der Katheter 2 sind in Aussparungen 12a der Form 12 Ausrichtelemente 16 einsetzbar, wobei jeweils ein Ausrichtelement 16 jedem Katheter 2 zugeordnet ist. Die zentrisch auf die Katheter 2 ausgerichteten Ausrichtelemente 16 greifen mit daran ausgebildeten, durch den Formraum 18 hindurchführenden Zapfen 17 in das Sackloch 9 im Endabschnitt 8B des jeweiligen Stopfens 3 ein. Hierdurch wird erreicht, daß das mit dem Stopfen 3 verschlossene Ende des Katheters 2 exakt in der Form ausgerichtet ist. Die Ausrichtelemente 16 können, wie beim Ausführungsbeispiel dargestellt, einzeln in der Form 12 angeordnet sein. Ebenso können die Ausrichtelemente 16 aber auch zu einem einzigen Bauteil zusammengefaßt sein, welches dann die entsprechende Zahl an Zapfen 17 aufweist.

Schließlich ist die Form 12 mit einem nach oben offenen Formraum 18 versehen, dessen Gestalt exakt der Gestalt des späteren Körpers entspricht. Damit die sich durch den Formraum 18 hindurch erstreckenden Katheter 2 nicht durchhängen, läßt sich von dem Ende 5 her in jeden Katheter 2 eine Zentrierstange 19 einführen. Auch die Zentrierstange 19 ist an ihrer Spitze mit einem als Verbindungselement 20 dienenden Zapfen versehen, der in das Sackloch 9 des Abschnitts 8A eingreift.

Zunächst werden in die Form 12 bei abgenommenem Klemmstück 14 die einzelnen Katheter 2 eingelegt, die an ihrem einen Ende 4 bereits mit den eingesetzten Stoffen 3 sowie den außen aufgesetzten Klemmringen 10 versehen sind. Auch die Zentrierstangen 19 sind zu diesem Zeitpunkt bereits in die Katheter 2 eingeführt und justieren die Stopfen 3 am Katheterende. Die Klemmringe 10 sind in axialer Richtung gegen den radial erweiterten Mittelabschnitt 7 des Stopfens 3 geschoben, so daß das Material des jeweiligen Katheters 2 zwischen Klemmring 10 und Stopfen 3 eingespannt wird. Hierdurch wird eine sehr gute axiale Fixierung des Stopfens 3 innerhalb des Katheters erreicht.

Die so vorbereiteten Katheter 2 werden dann in der in Fig. 3 gezeigten Lage in die Form 12 eingelegt, und an ihren Enden 4 durch formschlüssiges Eingreifen der Zapfen der Ausrichtelemente 1 6 in die Stopfen 3 zentriert. Anschließend wird das Klemmstück 14 auf den Grundkörper 13 der Form 12 aufgesetzt und beispielsweise verschraubt.

Sodann wird die vorbereitete Form 12 auf einer waagerecht ausgerichteten Platte in einem Umluftofen plaziert und die Form für ca. 15 min. auf eine Temperatur von etwa 150 °C vorgeheizt. In die so vorgeheizte Form 12 wird eine Teilmenge der flüssigen oder gelartigen Masse für das Grundmaterial eingegossen, bis der Formraum 18 so hoch mit dieser Masse angefüllt ist, daß Katheter 2 und Klemmringe 10 im wesentlichen davon eingeschlossen und auf diese Weise fixiert sind. Die auf diese Weise eine erste Lage bildende Masse härtet aus bzw. geliert nach etwa 10 min. bei einer Temperatur von 150 °C. Mit "Aushärten" ist hier der Abschluß des Gelierprozesses gemeint, nicht ein völliges Verhärten des Materials, welches vielmehr stets weich und flexibel bleibt.

In einem weiteren Verfahrensschritt wird anschließend die Gewebeeinlage 6 auf die Oberfläche der Masse aufgelegt und leicht angedrückt. Anschließend wird weitere Suspension in den Formraum 18 eingefüllt und auch diese Masse zum Gelieren gebracht, wodurch auch die Gewebeeinlage 16 in das Grundmaterial 1 des Körpers eingebettet wird. Schließlich wird die Form aus dem Ofen genommen und abgekühlt. Zur Entnahme des Körpers aus der Form 12 müssen zunächst die Ausrichtelemente 16 entfernt werden, vorzugsweise durch axiales Zurückziehen aus dem Formraum 18. Hierbei entstehen Löcher, in die abschließend auf 160 °C erhitztes Grundmaterial eingespritzt wird. Nach dessen Abkühlung ist der hergestellte Körper allseitig homogen geschlossen.

Zum Schließen der bei dem Zurückziehen der Ausrichtelemente 16 zwangsläufig entstehenden Löcher ist es alternativ auch möglich, die Ausrichtelemente 16 bereits dann aus der Form 12 herauszunehmen, nachdem die erste Lage der Masse ausgehärtet ist. Zu diesem Zeitpunkt sind die Katheter bereits vorläufig durch die unterhalb befindliche Masse fixiert, so daß Lageverschiebungen nicht mehr möglich sind. Es werden daher nach Einfüllen des ersten Teilvolumens der Masse zunächst die Ausrichtelemente 16 aus der Form 12 herausgenommen und jeweils durch einen Stopfen 21 ersetzt, der, anders als das Ausrichtelement 16, keine in den Formraum 18 hineinragenden Teile aufweist. Bei dem anschließenden Einfüllen des zweiten Anteiles der Masse füllen sich dann zugleich die beim Herausnehmen der Ausrichtelemente 16 im Formraum 18 entstandenen Löcher, so daß letztlich ein homogener Verschluß entsteht.

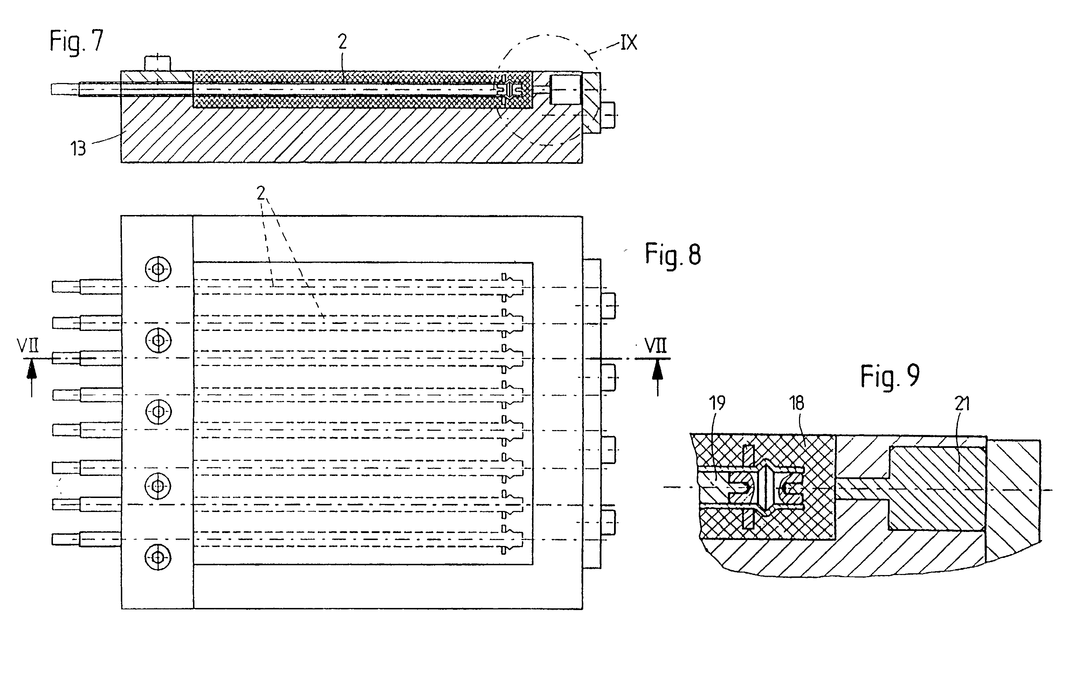

Die vollständig mit der Masse gefüllte Form ist in den Figuren 7 bis 9 dargestellt.

### Bezugszeichenliste

- 1: Grundmaterial
- 2: Katheter, Schlauch
- 3: Stopfen
- 4: geschlossenes Ende des Katheters
- 5: offenes Ende des Katheters
- 6: Gewebeeinlage
- 7: Mittelabschnitt des Stopfens
- 8A: Endabschnitt des Stopfens
- 8B: Endabschnitt des Stopfens
- 9: Sackloch
- 10: Klemmring
- 11: Bohrung
- 12: Form
- 12a: Aussparung der Form
- 13: Grundkörper
- 14: Klemmstück
- 15: Bohrung
- 16: Ausrichtelement
- 17: Zapfen
- 18: Formraum
- 19: Zentrierstange
- 20: Verbindungselement
- 21: Stopfen

## Patentansprüche

1. Verfahren zur Herstellung eines flexiblen Körpers mit darin eingebetteten Schläuchen oder Kathetern, insbesondere für die Strahlentherapie im Nachladeverfahren (After-Loading), bei dem die Schläuche oder Katheter (2) in eine Form (12) eingelegt und darin in definierter Lage und Ausrichtung fixiert werden, bevor zur Ausformung des Grundmaterials (1) des Körpers eine flüssige oder gelartige Masse in den Formraum (18) der Form eingefüllt wird und darin unter Einbettung der Schläuche oder Katheter (2) aushärtet,
**dadurch gekennzeichnet,**
**daß** die Schläuche oder Katheter (2) in der Weise in die Form (12) eingelegt werden, daß sich deren eine Enden (4) noch innerhalb des Formraums (18) der Form (12) befinden, daß diese Enden (4) dauerhaft verschlossen und daran vor Einfüllen der flüssigen oder gelartigen Masse Ausrichtelemente (16) angesetzt werden, die sich an der Form (12) abstützen.

2. Verfahren nach Anspruch 1, **dadurch gekennzeichnet, daß** die Enden (4) durch Einsetzen eines Stopfens (3) dauerhaft verschlossen werden.

3. Verfahren nach Anspruch 2, **dadurch gekennzeichnet, daß** zur exakten axialen Lagefixierung des Stopfens (3) dieser an dem zugehörigen Ausrichtelement (16) abgestützt wird.

4. Verfahren nach Anspruch 3, **dadurch gekennzeichnet, daß** über die Schläuche oder Katheter (2) Klemmringe (10) gezogen werden und daß die Klemmringe (10) axial gegen eine radiale Erweiterung (Mittelabschnitt 7) des Stopfens (3) geschoben werden, so daß Material des jeweiligen Schlauches bzw. Katheters zwischen Klemmring (10) und Stopfen (3) eingespannt wird.

5. Verfahren nach einem der vorangehenden Ansprüche, **dadurch gekennzeichnet, daß** nach dem Aushärten zumindest eines Teilvolumens der Masse die Ausrichtelemente (16) aus dem Grundmaterial (1) entfernt werden, und daß die bei dem Entfernen entstehenden Hohlräume verschlossen werden, vorzugsweise mit dem gleichen Material, aus dem die Masse besteht.

6. Verfahren nach einem der vorangehenden Ansprüche, **dadurch gekennzeichnet, daß** vor dem Einfüllen der flüssigen oder gelartigen Masse Zentrierstangen (19) von den nicht verschlossenen Enden (5) der Schläuche oder Katheter her in diese eingeführt werden, und daß die Zentrierstangen (19) nach dem Aushärten der Masse wieder gezogen werden.

7. Verfahren nach Anspruch 6, **dadurch gekennzeichnet, :daß** die Zentrierstangen (19) so weit in die Schläuche oder Katheter eingeführt werden, bis Verbindungselemente (20) an den Spitzen der Zentrierstange (19) formschlüssig in entsprechende Elemente (9) von Stopfen (3) eingreifen, die die Enden der Schläuche bzw. Katheter verschließen.

8. Flexibler Körper mit darin eingebetteten Schläuchen oder Kathetern, insbesondere für die Strahlentherapie im Nachladeverfahren (After-Loading), in dem sich die einen Enden (4) der Schläuche oder Katheter (2) innerhalb des Körpers befinden und dort durch in die Enden eingesetzte Stopfen (3) verschlossen sind, wobei die nach außen weisende Stirnfläche des Stopfens (3) durch das Grundmaterial (1) des flexiblen Körpers abgedeckt ist.

9. Flexibler Körper nach Anspruch 8, **dadurch gekennzeichnet, daß** die Stopfen (3) jeweils eine radiale Erweiterung (Mittelabschnitt 7) aufweisen, die sich innerhalb des Schlauches oder Katheters (2) befindet, und gegen die ein außen auf dem Schlauch oder Katheter (2) aufsitzender Klemmring (10) gespannt ist.

10. Flexibler Körper nach Anspruch 8 oder Anspruch 9, **dadurch gekennzeichnet, daß** der Stopfen (3) an seiner nach außen weisenden Stirnfläche mit einer Einsenkung (9) versehen ist, in die bei der Herstellung des Körpers ein Ausrichtelement eingreifen kann.

## Claims

1. Method of producing a flexible body with tubes or catheters embedded therein, in particular for radiation therapy in the after-loading method, in which the tubes or catheters (2) are placed in a mould (12) and fixed therein in a defined position and alignment before a liquid or gel-like compound is filled into the mould space (18) of the mould, in order to mould the basic material (1) of the body, and cured therein with embedding of the tubes or catheters (2), **characterized in that** the tubes or catheters (2) are placed in the mould (12) such that one of their ends (4) is still located within the mould space (18) of the mould (12), these ends (4) are permanently closed and alignment elements (16) which are supported on the mould (12) are attached thereto prior to filling of the liquid or gel-like compound.

2. Method according to Claim 1, **characterized in that** the ends (4) are permanently closed by inserting a stopper (3).

3. Method according to Claim 2, **characterized in that** in order to precisely fix the axial position of the stopper (3) the latter is supported on the associated alignment element (16).

4. Method according to Claim 3, **characterized in that** clamping rings (10) are drawn over the tubes or catheters (2) and **in that** the clamping rings (10) are pushed axially against a radial widening (middle section 7) of the stopper (3) so that material of the respective tube or catheter is clamped between clamping ring (10) and stopper (3).

5. Method according to any of the preceding claims, **characterized in that** the alignment elements (16) are removed from the basic material (1) after curing of at least a part-volume of the compound and **in that** the cavities that result upon the removal are closed, preferably using the same material as that of which the compound is composed.

6. Method according to any of the preceding claims, **characterized in that**, prior to filling of the liquid or gel-like compound, centring rods (19) are inserted into the tubes or catheters by way of the non-closed ends (5) thereof, and **in that** the centring rods (19) are removed again following curing of the compound.

7. Method according to Claim 6, **characterized in that** the centring rods (19) are inserted into the tubes or catheters until connection elements (20) at the tips of the centring rods (19) engage in a form-fitting manner in corresponding elements (9) of stoppers (3) which close the ends of the tubes or catheters.

8. Flexible body with tubes or catheters embedded therein, in particular for radiation therapy in the after-loading method, in which the one ends (4) of the tubes or catheters (2) are located within the body and are closed there by a stopper (3) inserted into the ends, where the outwardly pointing end face of the stopper (3) is covered by the basic material (1) of the flexible body.

9. Flexible body according to Claim 8, **characterized in that** the stoppers (3) in each case have a radial widening (middle section 7) which is located within the tube or catheter (2) and against which a clamping ring (10) that bears against the outside of the tube or catheter (2) is stressed.

10. Flexible body according to Claim 8 or Claim 9, **characterized in that** the stopper (3) is provided on its outwardly pointing end face with a recess (9) in which an alignment element can engage during production of the body.

## Revendications

1. Procédé de fabrication d'un corps flexible dans lequel des tubulures ou des cathéters sont noyés, notamment pour la radiothérapie dans le procédé à projection de source radioactive *(after-loading),* dans lequel les tubulures ou les cathéters (2) sont introduits dans un moule et fixés dans celui-ci dans une position et une orientation définies puis, pour former la matière de base (1) du corps, une masse liquide ou gélifiée est coulée dans la cavité (18) du moule où elle durcit en surmoulant les tubulures ou les cathéters (2),
**caractérisé en ce que**
les tubulures ou les cathéters (2) sont introduits dans le moule (12) de telle façon que les unes (4) de leurs extrémités se trouvent encore à l'intérieur de la cavité (18) du moule (12), **en ce que** ces extrémités (4) sont obturées en permanence et **en ce que**, avant la coulée de la masse liquide ou gélifiée, des éléments d'alignement (16) s'appuyant sur le moule (12) sont assujettis à ces extrémités.

2. Procédé selon la revendication 1, **caractérisé en ce que** les extrémités (4) sont obturées en permanence par la mise en place d'un bouchon (3).

3. Procédé selon la revendication 2, **caractérisé en ce que**, pour fixer exactement dans le sens axial la position du bouchon (3), celui-ci s'appuie sur l'élément d'alignement (16) correspondant.

4. Procédé selon la revendication 3, **caractérisé en ce que** des bagues de serrage (10) sont passées sur les tubulures ou les cathéters (2) et **en ce que** les bagues de serrage (10) sont poussées axialement contre une extension radiale (partie médiane 7) du bouchon (3), de telle façon que la matière de chaque tubulure ou cathéter est pincée entre la bague de serrage (10) et le bouchon (3).

5. Procédé selon l'une des revendications précédentes, **caractérisé en ce que**, après le durcissement d'au moins une partie du volume de la masse, on retire les éléments d'alignement (16) de la matière de base (1) et **en ce que** l'on obture les creux résultant de ce retrait, de préférence avec la même matière que celle dont la masse se compose.

6. Procédé selon l'une des revendications précédentes, **caractérisé en ce que**, avant la coulée de la masse liquide ou gélifiée, on introduit des tiges de centrage (19) dans les tubulures ou cathéters, par leurs extrémités (5) non obturées, et **en ce que** l'on retire les tiges de centrage (19) après le durcissement de la masse.

7. Procédé selon la revendication 6, **caractérisé en ce que** les tiges de centrage (19) sont introduites dans les tubulures ou cathéters jusqu'à ce que des éléments de liaison (20) à la pointe des tiges de centrage (19) s'engagent positivement dans des éléments correspondants (9) des bouchons (3) qui obturent les extrémités des tubulures ou cathéters.

8. Corps flexible dans lequel sont surmoulées des tubulures ou des cathéters, notamment pour le procédé à projection de sources radioactives *(after- loading),* dans lequel les unes des extrémités (4) des tubulures ou cathéters (2) se trouvent à l'intérieur du corps et sont obturées à cet endroit par des bouchons (3) introduits dans les extrémités, la face du bouchon (3) dirigée vers l'extérieur étant recouverte par la matière de base (1) du corps flexible.

9. Corps flexible selon la revendication 8, **caractérisé en ce que** les bouchons (3) présentent chacun une extension radiale (partie médiane 7) qui se trouve à l'intérieur de la tubulure ou du cathéter (2) et contre laquelle une bague de serrage (10) passée à l'extérieur sur la tubulure ou le cathéter (2) est bloquée.

10. Corps flexible selon la revendication 8 ou la revendication 9, **caractérisé en ce que** le bouchon (3) est muni, sur sa face dirigée vers l'extérieur, d'un lamage (9) dans lequel un élément d'alignement peut s'engager lors de la fabrication du corps.
